# EUROPEAN PATENT APPLICATION

(11) **EP 2 241 351 A1**
(43) Date of publication of application: **20.10.2010**
(21) Application number: 09305325.4
(22) Date of filing: 16.04.2009
(51) Int. Cl.: A61P 17/16, A61K 31/01, A61K 31/047, A61K 45/06, A61K 9/00, A61Q 19/00

(54) **Pharmaceutical composition comprising glycerol, white soft paraffin and liquid paraffin for the treatment of uremic xerosis**

(71) Applicant: ORFAGEN, 31520 Ramonville Saint Agne (FR)
(72) Inventor: Dupuy, Patrick, 31000 Toulouse (FR)
(74) Representative: Warcoin, Jacques

(57) **Abstract**

The invention relates to a pharmaceutical composition comprising a combination of glycerol, white soft paraffin and liquid paraffin as active ingredients for topical use to treat uremic xerosis.

## Description

The invention relates to the treatment of uremic xerosis.

Patients undergoing maintenance renal dialysis (MRD) present with several dermatologic complications. Among these complications, xerosis (rough and scaly skin, also called uremic xerosis) and pruritus (also called uremic pruritus) are the most common manifestations. Both of these non specific symptoms are also observed in patients with chronic renal failure before the need of dialysis, but are significantly increased when patients initiate MRD, either haemodialysis or peritoneal dialysis. They are classically absent in acute renal failure, and are not correlated with the plasmatic levels of urea. Furthermore, they typically disappear after renal transplantation.

Uremic xerosis is a very common complaint in patients who undertake MRD. According to the literature, it is affecting 50% to 85% of the patients whereas 30 to 40% of patients report this symptom before starting MRD or after renal transplant. Age is an aggravating factor. It often affects all the body, and is usually more severe in some areas (legs, forearms, hands, back). In large series, the intensity of xerosis has been described as mild in 30-40%, moderate in 35-50%, and severe in 15-30% of the MRD patients. It is a chronic syndrome, with a clinical picture comprising a dry skin appearance, marked scaling and roughness, and a poor turgor (i.e. failure to the skin to reassume prompt normal contour, once the skin has been pulled upward) of the skin. The negative influence of climate and environmental factors (wind, cold, low humidity) is frequently reported. Associated signs are premature skin ageing (elastosis) and pruritus.

Uremic xerosis is considered to be an important factor contributing to uremic pruritus. Like in xerosis, pruritus frequency in MRD patients is greatly increased, ranging from 35% to 65%, whereas it is observed in about 30% of patients with chronic renal failure without MRD. The frequency of uremic pruritus is higher in patients with xerosis than patients without. Based on large published series, xerosis of moderate to severe intensity leads to a 50-100% increase in uremic pruritus. Similarly, uremic pruritus severity appears to be directly related to xerosis severity : the more intense is xerosis, the greater the intensity of pruritus is.

By contrast, it is not significantly influenced by factors such as patient age, sex, underlying renal disease or type of dialyzate used for haemodialysis (acetate-based or bicarbonate-based), except for haemodialyzed patients using less permeable dialysis membranes (polysulphone), who may have a higher incidence of pruritus than patients dialyzed with more permeable (hemophane or cuprophane) membranes. Like uremic xerosis, uremic pruritus is a chronic symptom with, sometimes, periods of exacerbation during or soon after haemodialysis. It may be generalized or localized. Especially when associated with xerosis, pruritus provokes vigorous scratching, which can produce extensive excoriations, lichenification, prurigo nodularis (multiple brown nodules) and sometimes cutaneous superinfections (secondary impetigo). Bothersome disturbance of sleeping and daily activities are common. The pathophysiology of uremic pruritus is not understood and appears multifactorial. Among likely factors, uremic xerosis has been considered as a main factor.

The cause of uremic xerosis in MRD patients is unclear. Multiple factors may contribute towards the rough and scaly skin appearance of MRD patients, including skin dehydration and reduced sebum and sweat excretion. Another possible explanation is an alteration in the metabolism of vitamin A, which is in increased concentration in uremic patients. Xerotic skin of hypervitaminosis A syndrome resembles the skin lesions of xerotic patients with MRD. Atrophy of sebaceous glands, resulting in lower levels of surface lipids of the skin, may also participate in the pathogenesis of uremic xerosis. Other potential factors include thyroid underactivity and skin inflammation by the increased number of cutaneous mast cells releasing histamine.

The primary measure for treatment of uremic xerosis in MRD patients remains the generous use of topical emollients. Demonstration of its efficacy has never been shown but is universally accepted by therapists. Moisturizing emollients have both rehydrating and occlusive properties, with optimal effects when used on a regular basis, i.e. at least twice a day in a continuous course.

A first problem with a treatment based on emollient products is that in the cosmetic market they comprise an unlimited number of non-active ingredients, including fragrance materials. This exposure to an excessive number of ingredients is potentially a higher risk for MRD patients to develop local intolerance than it might be for healthy individuals.

Another problem with a treatment based on emollient products is the cost of emollients. This is possibly a factor of poor compliance for patients with MRD. Conditions of efficacy of an emollient include generous and repetitive applications of the product every day on xerotic areas, i.e. over a large area of the body surface. Treatment is long-lasting. Available products are registered under the cosmetic regulations in most countries, including the European Community. None of them has been approved in the proposed indication, and a few of them have been marketed as ethical products in other indications. Nonetheless, MRD patients have a medical need for these products, and they cannot be provided to them other than through the cosmetic market. Consequently, products are only available to patients at full cost. The material to apply should be large, about 1 mg/cm², to avoid incomplete coverage, resulting in high consumption of the product in normally compliant patients. For instance, this would require a consumption of about 250 ml/week in adult patients for total coverage (total body surface area (BSA) : 1.70m², surface application: 50% BSA, 2mg/cm² to apply, 2 applications/day, product density ≈ 1000). Providing that the approximate cost of an emollient in the European cosmetic market is around 10 Euros for 100 ml of product, the expense could be estimated monthly up to 100 Euros for an adult. Without substantial reimbursement of the product, it is obvious that the effective cost of emollients is very likely to alter their optimum use by patients affected by the proposed indication.

Thus, there exist important needs for an emollient treatment method, approved as a medicinal product, in uremic xerosis showing clinical efficacy in normalizing uremic xerosis, and better compliance and better tolerance by the target population.

For this purpose, the present inventors have first shown that an emollient based on glycerol and paraffin shows a good occlusive effect due to a synergistic action of glycerol and paraffin.

Second, the present inventors performed a randomized, placebo-controlled, double-blind study showing therapeutic efficacy of an emollient based on glycerol and paraffin on uremic xerosis.

Patients undergoing MRD are advantageously concerned by a treatment of uremic xerosis according to the invention.

Therefore a first object of the invention relates to a pharmaceutical composition for topical use comprising a combination of glycerol, white soft paraffin and liquid paraffin as active ingredients for use to treat uremic xerosis.

Patients undergoing maintenance renal dialysis (MRD) are advantageously concerned by the treatment of uremix xerosis according to the invention.

Within the meaning of the present invention, « active association» means the association of glycerol, white soft paraffin and liquid paraffin.

Advantageously, white soft paraffin and liquid paraffin are controlled according to respectively monograph n°0496, Monograph n°1799 and Monograph n°0239 of the « European Pharmacopeia », 6th Edition.

Advantageously, white soft paraffin of the active has a dropping point between 35 and 70°C, preferably between 51 and 57°C, more preferably of approximately 54°C. The dropping point is measured according to the process 2.2.17 described in « European Pharmacopeia », 6eme Edition.

Advantageously, white soft paraffin of the active association has a consistency between 175 and 195 1/10 mm, preferably about 185 1/10 mm (cone penetration at 25°C).

Advantageously, white soft paraffin of the active association has a viscosity between 4 and 5 cSt at 100°C, preferably about 4.8 cSt at 100°C.

The active association is present in the composition according to the invention, in a proportion between 10 and 50 % and preferably between 20 and 30 % w/w compared to the total weight of the composition ; the concentration of glycerol is comprised between 5 and 30 %, preferably between 10 and 20 % and more preferably is about 15% w/w compared to the total weight of the composition, the concentration of white soft paraffin is comprised between 3 and 20 %, preferably between 5 and 10 % and more preferably is about 8% w/w compared to the total weight of the composition and the concentration of liquid paraffin is comprised between 0.5 and 5 %, preferably between 1 and 3 % and more preferably is about 2% w/w compared to the total weight of the composition.

Water is comprised between 30 and 80 w/w compared to the total weight of the composition.

Advantageously, the composition according to the invention comprises about 15% of glycerol, about 8% of white soft paraffin and about 2% of liquid paraffin w/w compared to the total weight of the composition.

The dermatological composition according to the invention comprises furthermore usual dermatologically acceptable excipients.

Preferably, the dermatological composition according to the invention is a water-in-oil (W/O) or oil-in-water (O/W) emulsion, a water-in-oil-in-water (W/O/W) or oil-in-water-in-oil (O/W/O) emulsion, or a hydrodispersion or a lipodispersion, a gel or an aerosol, more preferably a water-in-oil (W/O) or a oil-in-water (O/W) emulsion, and the most preferably a oil-in-water (O/W) emulsion.

Indeed, the type of formulation (neat substances, solutions and emulsions) and the type of emulsion (W/O or O/W emulsion) intervene in the activity of the active association.

When used as neat substances, white soft paraffin had no occlusive activity when applied in powder form ¹, but a highly occlusive effect when applied in an oil phase (liquid state). This indicates that paraffin shows occlusive properties when applied uniformly on the skin surface. In order to apply white soft paraffin uniformly, a liquid state of white soft paraffin is required, e.g. as formulated in emulsions. Paraffin in emulsions is more occlusive than in solutions ².

The hydrating and occlusive effects of emulsions themselves vary according to their types (w/o and o/w). The w/o formulation with white soft paraffin even at high dosage (46.75%) is able to induce a significant increase in stratum corneum (SC) () water content, but has limited or no significant effect on skin barrier function by TransEpidermal Water Loss (TEWL) reduction ². Similarly, o/w emulsions by themselves have a limited effect on skin hydration, or even deleterious effects on the SC barrier function ³. When associated with liquid paraffin, the o/w emulsion looses water faster than the w/o emulsion, and begins to show earlier but milder occlusivity than the w/o emulsion ¹. By contrast, the adjunction of glycerol to paraffin in an o/w formulation can prevent SLS (Sodium Laurylsulphate)-induced skin dehydration ³.. Using the same SLS model, Grunewald and co-workers showed a similar synergistic effect between a o/w emulsion and glycerol as regards to prevention of skin dehydration, compared to urea in the same o/w formulation and other w/o commercial formulations ⁴. Glycerol in o/w emulsions acts through a long-term regenerative process of the disrupted SC, rather than a simple physicochemical mechanism of action such as hydration ^{5,6,7}.

In conclusion, on the one hand, o/w emulsions without glycerol have limited or transitory effects on the moisture of the SC a well as possible deleterious effects on the skin barrier, whereas the w/o emulsions exert a better hydration profile but a limited barrier effect. On the other hand, whereas w/o emulsions act merely as a protective film that induces skin hydration, o/w emulsions facilitate the hydrating and regenerative effects of glycerol.

Preferably, 90% of the paraffin droplets present in a composition according to the invention in the form of an emulsion have a size below 30 µm, more preferably 25 µm

Indeed, the occlusive properties of white soft paraffin in emulsions also vary with the size of the droplets they constitute in the formulation ¹. The smaller the paraffin particles are, the more occlusive white soft paraffin is, with an optimum threshold of activity being reached with droplet size under 30 µm. An internal stereomicroscopic study showed that more than 90% of the paraffin droplets dispersed in Composition A, Example 1, have a size below 25 µm. This indicates that the paraffin dispersion in the proposed formulation provides optimum bioavailability of this active compound.

Dermatologically acceptable excipients can be any excipient among those well-known by the one skilled in the art in order to obtain a composition for topical application in the form of a cream, a lotion, a gel, an ointment, an emulsion, a microemulsion, a spray, etc

The composition according to the invention can in particular comprise additives, as emulsifying agents, consistency agents, gelling agents, water-binding agents, spreading agents, stabilizing agents, colouring agents, perfumes and preservatives.

Appropriate emulsifying agents comprise stearic acid, trolamine, lanolin alcohols (e.g. Amerchol L101).

Preferably, appropriate emulsifying agents do not have disruptive effects on the SC and, thus, do not modify the barrier function. The one skilled in the art will chose appropriate emulsifying agents according to his general knowledge. For example, stearic acid has been shown to induce no alteration on normal skin and SLS-modified skin in humans, at the concentration of 5% ⁸.

Preferably, the composition according to the invention comprises about 5% of emulsifying agents w/w compared to the total weight of the composition.

Advantageously, the composition according to the invention comprises between 1 and 5% of stearic acid, preferably about 3% w/w compared to the total weight of the composition.

Advantageously, the composition according to the invention comprises between 0 and 2% of trolamine, preferably about 0.5% w/w compared to the total weight of the composition.

Advantageously, the composition according to the invention comprises between 0 and 5% of lanolin alcohols, preferably about 2% w/w compared to the total weight of the composition.

Appropriate consistency agents comprise glycerol monostearate and PEG.

Preferably, the composition according to the invention comprises about 5% of consistency agents w/w compared to the total weight of the composition.

Advantageously, the composition according to the invention comprises between 2 and 10% of glycerol monostearate, preferably about 5% w/w compared to the total weight of the composition.

Appropriate preservatives comprise methyl parahydroxybenzoate, propyl parahydroxybenzoate and chlorocresol.

Preferably, the composition according to the invention comprises about 0.1 % of preservatives w/w compared to the total weight of the composition.

Advantageously, the composition according to the invention comprises between 0 and 1% of methyl parahydroxybenzoate, preferably about 0.09% w/w compared to the total weight of the composition.

Advantageously, the composition according to the invention comprises between 0.05 and 1% of propyl parahydroxybenzoate, preferably about 0.1% w/w compared to the total weight of the composition.

Appropriate spreading agents comprise dimethicone and polydimethylcyclosiloxane.

Preferably, the composition according to the invention comprises about 2% of spreading agents w/w compared to the total weight of the composition.

Advantageously, the composition according to the invention comprises between 0 and 2% of dimethicone, preferably about 0.5% w/w compared to the total weight of the composition.

Advantageously, the composition according to the invention comprises between 1 and 3% of polydimethylcyclosiloxane, preferably about 2.5% w/w compared to the total weight of the composition.

Appropriate water-binding agents comprise polyethylene glycol, preferably polyethylene glycol 600.

Preferably, the composition according to the invention comprises about 8% of water-binding agents w/w compared to the total weight of the composition.

Advantageously, the composition according to the invention comprises between 2 and 10% of polyethylene glycol, preferably about 5% w/w compared to the total weight of the composition.

Water used in the aqueous phase of the emulsion can be distilled water or thermal water with dermato-cosmetic properties.

Advantageously, the composition according to the invention comprises :
- about 15 % of glycerol,
- about 8% of white soft paraffin,
- about 2% of liquid paraffin,
- about 1 at 5% of stearic acid,
- about 2 at 10% of glycerol monostearate,
- about 1 at 3% of polydimethylcyclosiloxane,
- about 0.2 at 2% of dimethicone,
- about 2 at 10% of polyethylene glycol 600,
- about 0 to 2% de trolamine,
- about 0.05 to 1% de propyl parahydroxybenzoate
- water q.s..

A second object of the invention relates to the use of a composition according to the invention for the preparation of a medicament to treat uremic xerosis

A third object of the invention relates to the use of a composition comprising a combination of glycerol, liquid and solid paraffin as active ingredients for the preparation of a medicament to treat uremic xerosis.

The invention will further be illustrated in view of the following figures and examples.

### FIGURES :

Figure 1: Corneometry - Mean % of evolution in relation to baseline values
Figure 2: TEWL - Mean % of evolution in relation to baseline values
Figure 3: Scaling measurements of uremic xerosis on the lower legs - ITT population
Figure 4: Uremic pruritus of the lower legs treated by active or vehicle- ITT population
Figure 5: Severity of uremic xerosis of the lower leg initially treated by active, the lower leg initially treated by vehicle, the chest and the forearm during study course - ITT population

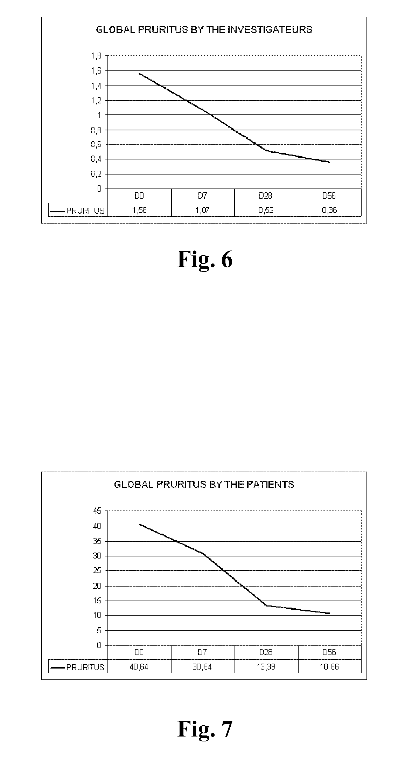

Figure 6: Global uremic pruritus assessed by the investigators during the study course - ITT population
Figure 7: Global uremic pruritus assessed by the patients (100 mm VAS) during the study course - ITT population

### EXAMPLES

### Example 1 : Formulation

### Composition A

- 15 g of glycerol,
- 8 g of white soft paraffin
- 2 g of liquid paraffin,
- and as excipients stearic acid, glycerol monostearate, polydimethylcyclosiloxane, polyethylene glycol (PEG) 600, methyl parahydroxybenzoate, propyl parahydroxybenzoate, dimethicone, trolamine, Amerchol L101 ®,
- water q.s. 100 g.

### Composition B

- 15 g of glycerol,
- 8 g of white soft paraffin
- 2 g of liquid paraffin,
- and as excipients stearic acid, glycerol monostearate, polydimethylcyclosiloxane, polyethylene glycol (PEG) 600, propyl parahydroxybenzoate, dimethicone, trolamine,
- water q.s. 100 g.

### Example 2 : Study of the separate and combined contribution of the active ingredients glycerol and paraffin in the Example 1 formulation to the restoration of skin hydration and barrier function

A total of 55 healthy volunteers (age range: 18-40 years) with a common xerosis of mild to moderate severity (Kligman's score: 2 or 3) were enrolled in a comparative, randomised, double-blind study. Glycerol alone (15%), paraffin alone (10%), their combination (Composition A) and their vehicle were tested in each individual as a single skin application in a thin layer (1 finger tip unit, i.e. corresponding to about I gr of product) on their randomly allocated sites of the forearms (2 test treatments by forearm). Randomisation to test sites was performed using a Latin square procedure. Skin water content was measured by corneometry using the CM 825 apparatus (Courage and Khazaka) and skin barrier function was evaluated by TEWL, using evaporimetry (Courage and Khazaka), up to 12 hr after the test treatment application (baseline, 1 hr, 2 hr, 4 hr, 8 hr, 10 hr and 12 hr). Instrumental measurements were made in controlled environmental conditions (temperature: 22 ± 2°C; relative humidity: 50 ± 10%). The local tolerance of the test products was evaluated by a dermatologist at the end of the study (12 hr). The intended analysis plan was an analysis of variance in cross-over.

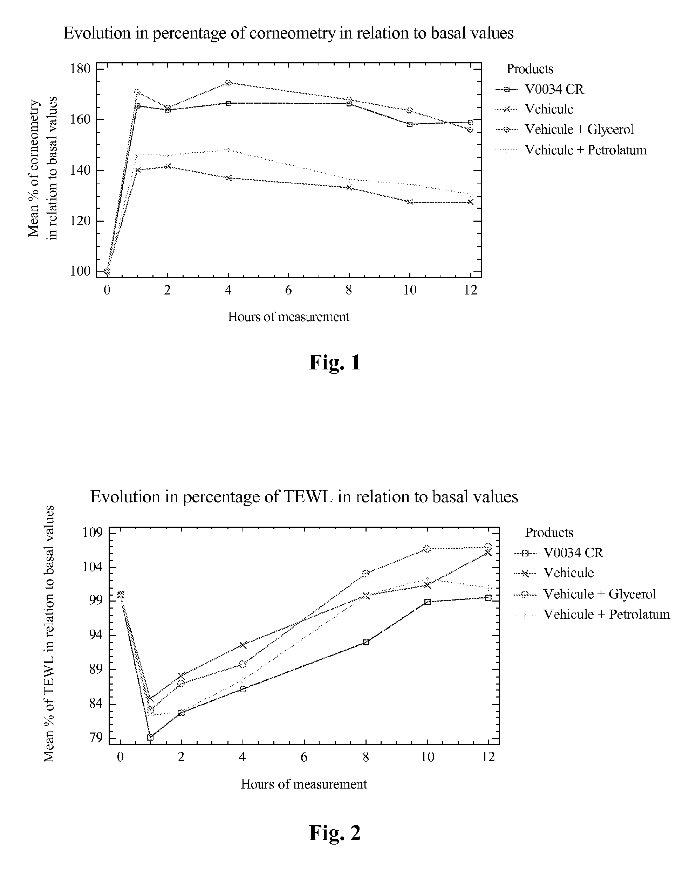

Among the 55 randomised subjects, 4 subjects were not included in the statistical analysis for major protocol violations. Thus, the population studied for efficacy comprised 51 volunteers (all-patients-treated or APT population). After opening the randomisation code, two volunteers were found to have aberrant TEWL values, which were considered related to technical problems. Instead, differences between treatments were determined by the analysis of variance (ANOVA) with repeated measures, adjusted by the two following multiple pair wise comparison tests: the Bonferroni *test* and the Dunnett test. For completion, results in both APT populations (restricted APT *population* of 49 volunteers, full APT population of 51 patients) are presented below. Results on skin water content in the restricted APT population are illustrated in **Figure 1****.** Comparison of baseline values showed no difference between treatment groups in both populations (restricted APT, full APT). In the restricted APT population analysis, treatment with vehicle alone resulted in an increase in SC hydration by about 40% after 1 hr, that progressively decreased up to 12 hr. Paraffin had a mild hydrating effect compared to vehicle, although significant (p<0.03). Conversely, glycerol induced a dramatic increase in water content by 65% compared to baseline, and this increase was maintained all over the observation period (12 hr). Difference between the glycerol treatment group and the vehicle group was statistically different (p<0.0001, Bonferroni test). Similarly, the combination glycerol and paraffin resulted in a high skin hydration that was comparable to glycerol alone (p<0.0001 compared to vehicle, Bonferroni test; confirmed by Dunnett test: p=0.0001). In the full APT population analysis, results and statistical data were similar to those obtained in the restricted APT population analysis, except for paraffin, which did not show significant hydrating effect compared to vehicle. Accordingly, the hydration capacity of the Composition A was mainly attributed to glycerol.

Results on TEWL in the restricted APT population are illustrated in **Figure 2****.** TEWL values between treatment groups were similar at baseline in both APT populations. In the restricted APT population analysis, vehicle alone induced a decrease in TEWL up to 8 hr, that was maximum (15% reduction) after 1 hr. Glycerol alone did not significantly modify TEWL compared to vehicle. Paraffin alone was slightly but significantly more efficient than vehicle on TEWL reduction (p<0.02, Bonferroni test). Only the combination glycerol and paraffin resulted in a higher (about 20% reduction from baseline after 1 hr) and more lasting (up to 10 hr) decrease in TEWL, compared to vehicle (p<0.002, Bonferroni test; confirmed by Dunnett test: p=0.0009). In the full APT analysis, results and statistical data were comparable to the restricted APT population analysis, except that paraffin alone showed no significant effect compared to vehicle.

In conclusion, the occlusive capacity of Composition A was mainly exerted by the combination of glycerol and paraffin, whereas each active compound alone provided no (glycerol) or limited (paraffin) effects. Thus, the adjunction of glycerol to paraffin increases the occlusive effects of paraffin.

It was concluded that the proposed medicinal product had both a significant hydrating effect and an occlusive effect.

Glycerol alone showed a hydrating effect but no action on TEWL, and paraffin alone showed a limited effect on TEWL but no effect on skin hydration.

Only the combination of the two compounds exerted at best both hydrating and occlusive effects, indicating a synergistic action between glycerol and paraffin.

### Example 3: clinical studies

### Study population

One hundred (100) patients were enrolled in 4 participating centres (25/centre) according to the following inclusion criteria. The study population was to include male and female patients of at least 10 years of age, undergoing MRD (haemodialysis or peritoneal dialysis) due to end-stage renal disease (ESRD). These patients presented with uremic xerosis related to their ESRD status. Uremic xerosis was moderate to severe, i.e. with a severity score of 2 to 4, and spread onto two symmetric areas of the lower legs of equal severity. Uremic xerosis was associated or not with uremic pruritus.

Patients with a known allergy to one of the test ingredients, and those having an intercurrent condition which might have interfered with a good conduct of the study were excluded. Patients treated with any moisturizing or emollient topical preparation within 7 days prior to study entry, with an instable dosage schedule of antipruritics (e.g. antihistamines, cholestyramine, opioid inhibitors, charcoal) within 4 weeks prior to study entry, and patients with phototherapy within 8 weeks prior to study entry were also excluded.

No paediatric patient was finally included, as the participating centres were referral centres for adults only. Upon request of the local ethics committee, children were excluded from the enrolled population in one centre (France).

One randomised patient withdrew secondarily his consent the day after baseline (day 1) and never applied the test products. Accordingly, this patient was excluded from the analysis, and 99 randomised patients (53 males, 46 females, with a median age of 65 years) who received at least one application of the test products, were included in the intent-to-treat population (ITT). Ninety-five (95) patients with no major deviation for the primary efficacy parameter were included in the per-protocol (PP) population. Eighteen (18) patients were prematurely withdrawn from the study, due to adverse event, own decision of the patient, insufficient response, or no comprehension of the protocol.

The demographic features (age and gender) of the ITT study population are presented in **Table 1.**

**Table 1: Demographic characteristics - ITT population**

| **ITT population** | **N = 99** |
|---|---|
| **Age (years)** | |
| Mean (sem) | 63.16(1.28) |
| Min / max | 18 / 89 |
| median | 65 |
| **Gender** | |
| Male | 53 ( 53.5% ) |
| female | 46 ( 46.5% ) |

The MRD technique performed in the ITT population was haemodialysis in 90 patients (91 %) and peritoneal dialysis in 9 patients (9%). The mean duration of MRD was 7.91 years (SEM: ± 0.71; median: 5; range: 1-34).

The underlying diseases resulting in MRD and their frequencies in the study population are given in **Table 2.**

**Table 2: Nature and frequency of the underlying diseases resulting in MRD at baseline - ITT population**

| **Underlying diseases** | **N = 99** |
|---|---|
| Glomerular diseases | 25 (25.25%) |
| Diabetic nephropathy | 15 (15.15%) |
| Nephrosclerosis | 15 (15.15%) |
| Polycystic kidney disease | 14(14.14%) |
| Congenital nephropathies | 1(1.01%) |
| Pyelonephritis | 5 (5.05% ) |
| Hypertensive nephropathy | 7 (7.07% ) |
| Other diseases | 9 (9.09%) |
| *Unknown* | *10(10.1%)* |

The mean duration of uremic xerosis at baseline was 5.40 years (SEM: ± 0.53; median: 4; range: 1-31; missing data for 3 patients). The patient distribution of severity of uremic xerosis on lower legs at baseline is given in **Table 3.** All patients presented with identical severity of uremic xerosis on each side of the lower legs, except for one patient whose uremic xerosis was severe on the lower leg treated by active and very severe on the lower leg treated by vehicle. Overall, uremic xerosis severity on both lower legs was mild in 2 patients (2%), moderate in 50 patients (51%), and severe to very severe in 47 patients (48%).

**Table 3: Distribution of severity of uremic xerosis on the lower legs at baseline - ITT population**

| **Lower legs** | **Score¹** | | | | |
|---|---|---|---|---|---|
| | | | **0 1 2 3** | | **4** |
| **Treated by active** | **1** | **1** | **50** | **36** | **11** |
| **Treated by vehicle** | **1** | **1** | **50** | **35** | **12** |

| | | | | | |
|---|---|---|---|---|---|
| ¹ 0 = smooth skin; 1 = patches of fine, powdery scales; 2 = diffuse ashy appearance with many fine scales; 3 = moderate scaling with beginning cracks; 4 = intense scaling, moderate cracks. | | | | | |

A total of 74 patients (75%) had a history of uremic pruritus. Among them, the mean duration of uremic pruritus was 4.81 years (SEM ± 0.52; median: 3; range: 1-22). Distribution of severity of global uremic pruritus at baseline in the ITT population is given in **Table 4.** Overall, 72 patients (73.5%) were reported by the investigators as having global uremic pruritus. The disease was mild in 19 patients (19.39%), moderate in 32 patients (32.65%), severe in 13 patients (13.27%) and very severe in 8 patients (8.16%).

**Table 4: Global uremic pruritus assessed by the investigators at baseline - ITT population**

| **Score** | **N = 98*** |
|---|---|
| No itch | 26 |
| Pruritus without the need of scratch | 19 |
| Pruritus with scratch, without excoriation | 32 |
| Pruritus with phys signs of excoriations | 13 |
| Totally restless pruritus | 8 |

| | |
|---|---|
| * missing data at baseline for one patient | |

### Study design

The study was multicentric and organised into 2 subsequent phases. Phase I (baseline to day 7) was a comparative period evaluating the test product (Composition A) versus its vehicle intra-individually (left lower leg vs. right lower leg comparison). This phase was randomised and double-blind. Phase II (days 7 to 56) was a non-comparative open-labelled period evaluating the test product alone.

In phase I, patients applied twice daily each test product onto one randomly assigned lower leg for 7 days. The other xerotic areas of the body were asked not to be treated. In phase II, Composition A was applied twice daily onto all xerotic and pruritic areas of the body for 49 days. The amount of products to apply depended on the surface area of the lesions (1mg/cm²) and patients were informed about the dose to use for each treated areas in terms of finger tips, one finger tip corresponding approximately to 1 g of product. For optimising the amount of product to apply, a table recommending the number of finger tips of product on each body area was given to the patients.

Study visits were performed at baseline, day 7, day 28 and day 56. To minimise inter-observer variation in end-point measurements, each patient was evaluated by the same physician at all visits.

### Evaluation parameters

All the evaluations were blinded to treatment allocation.

The primary efficacy criteria was the severity of uremic xerosis on the two symmetrical test areas of the lower legs at the end of phase I (day 7). This endpoint was evaluated by the investigators, using the defined 5-point El Gammal severity scale (0 = smooth skin; 1 = patches of fine, powdery scales; 2 = diffuse ashy appearance with many fine scales; 3 = moderate scaling with beginning cracks; 4 = intense scaling, moderate cracks). A therapeutic response was defined as a decrease of at least 2 grades of the xerosis at the end of the comparative phase (day 7).

The study included the following secondary efficacy parameters:
- The severity of uremic xerosis was evaluated on 4-test sites (forearm without the arteriovenous shunt, chest, the two lower legs) by the investigators using the 5-point severity scale, at all visits (baseline, day 7, day 28, day 56).
- A test side preference on the lower legs was performed at day 7 by the investigators according to a 3-point scale: 0 = right side comparable to left side; 1 = right side better than left side; 2 = left side better than right side.
- Scaling on both lower legs was analysed at baseline, day 7 and day 56 with four instrumental measurements (D-Squame®): SURFT, which evaluates the extension of the scales; MOD and SMOD, which determine the scale thickness with or without applying a detection threshold; and HI, which indicates the heterogeneity of the scales as regards to thickness.
- The severity of uremic pruritus on each lower leg was evaluated by the patients at baseline and day 7, using a 100-mm visual analog scale (VAS).
- The severity of global uremic pruritus was reported at all study visits by patients using a 100-mm VAS, and by the investigators using a modified 5-point scale as defined by De Marchi et al.^{18,19} This scale was graded as follows: 0 = no itch; 1 = pruritus without the need of scratch; 2 = pruritus with the need of scratch, but without physical signs of excoriation; 3 = pruritus accompanied by physical signs of excoriations; 4 = totally restless pruritus, interfering with work or activities of daily living or sleep.
- Patients assessed life quality at baseline and day 56 according to questionnaires using two scales, i.e. the Dermatologic Life Quality Index or DLQI²⁰ and the short-form (SF)-12 scale. DLQI measured the impact of dermatological diseases on patients' quality of life, whereas SF-12 is a multipurpose generic measure of health status. The SF-12 comprises 2 components, the Physical Component Summary (PCS-12) and the Mental Component Summary (MCS-12).
- Global tolerance was evaluated at day 56 by the investigators, using a 4-point scale graded as follows: 1 = very good tolerance, i.e. no functional or physical sign of local side effects; 2 = good tolerance, i.e. transitory functional signs, without physical sign nor necessary modification of the frequency of test application; 3 = poor tolerance, i.e. persisting functional signs or physical signs of local side effects, leading to modification of the frequency of the test application but no treatment discontinuation; 4 = very poor tolerance, i.e. functional and/or physical signs leading to treatment discontinuation.
- Patients graded their global agreement of the test products on efficacy, local tolerance and ease of use at day 56, according to the 4-point scale: 1 = very satisfactory, 2 = satisfactory, 3 = poorly satisfactory, 4 = not satisfactory at all.

In addition, physical examination was performed at baseline and end of study, and adverse events were recorded all over the study. The records of adverse events described the nature (diagnosis, signs and symptoms), severity, date/time of onset, date/time of end, outcome and actions taken, and relationship to study treatment according to the Investigator's opinion, as follows: unrelated, remote, probable.

Initial and final severity of adverse or intercurrent events was graded as mild (awareness of signs of symptoms, but easily tolerated), moderate (uncomfortable enough to cause interference with usual activity), or severe (incapacity with inability to work or do usual activity).

It was specified whether the event was serious (e.g. leading to hospitalisation) or not. Serious adverse events had to be notified within 2 working days to the sponsor and required specific report procedures.

### Data analysis

Statistical analysis of the primary efficacy parameter was made with the McNemar's test. The secondary efficacy qualitative parameters were evaluated by the Wilcoxon signed rank test or the sign test for the test side preference, and the quantitative parameters by the non parametric Wilcoxon test. A descriptive analysis was given for tolerance and adverse events.

### Results

### Comparative data

At baseline, severity of uremic xerosis was comparable between treatment groups, i.e. lower leg treated by active versus lower leg treated by vehicle, as difference between groups was not statistically significant. At day 7, treatment response (i.e. a decrease of at least 2 grades of the xerosis) was observed in 72 patients (73%) on the leg treated by the active and in 44 patients (44%) on the leg treated by the vehicle (**Table 5**). Difference between treatment groups was statistically significant (p<0.0001, Mc Nemar).

**Table 5: Patient distribution according to treatment response of uremic xerosis at day 7 - ITT population**

| | **Response** | **No response** |
|---|---|---|
| **Lower legs treated by active** | **72(73%)** | **27(27%)** |
| **Lower legs treated by vehicle** | **44 ( 44% )** | **55(56%)** |

Similarly, test side preference was in favour of the active group (N = 60, 60.6%), whereas the vehicle was found to be better in 13 patients (13.1 %) and comparable to active in 26 patients (26.3%, p<0.0001, sign test).
Scaling measurements by D-Squame® revealed comparable baseline values in all parameters. At day 7, Composition A was found to be significantly more efficient than the vehicle for 2 of the 4 studied parameters (SURFT and MOD parameters; p<0.0001, Wilcoxon test), whereas no statistical significant difference was observed for the parameters SMOD and HI between the two treatments, as shown in **Figure 1****.** At baseline, severity of uremic pruritus of the lower legs by the patients was comparable between the active group (mean VAS ± SEM: 31.19 ± 3.11 mm, median: 22, range 0-100) and the vehicle group (mean ± SEM: 31.23 ± 3.16 mm, median: 20, range 0-99.5). Uremic pruritus at day 7 was significantly improved from baseline by around 40% in each treatment group (p<0.0001, Wilcoxon, intragroup analysis), as shown in **Figure 2****.** However, no difference could be demonstrated between the two groups (intergroup analysis).

### Non comparative data

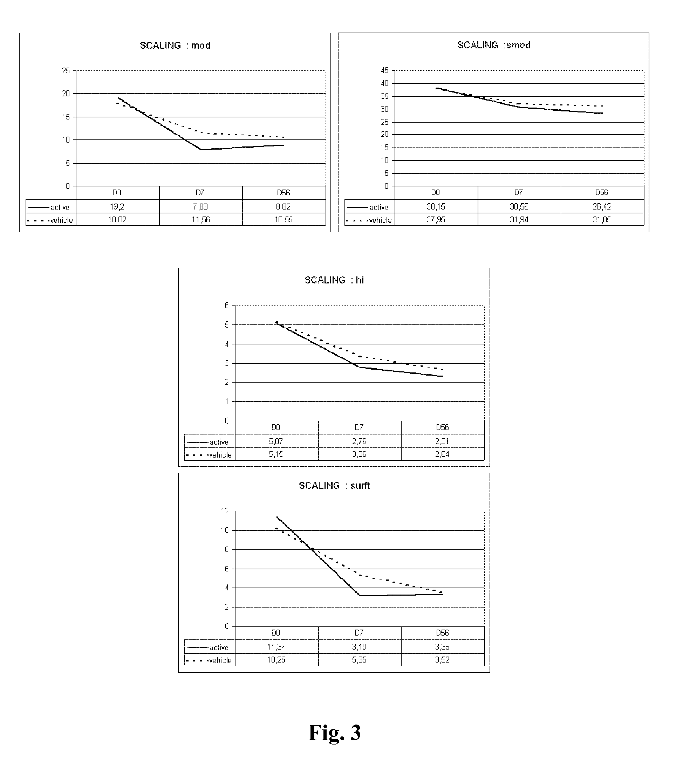

From day 7 to day 56, Composition A further reduced the severity of uremic xerosis on the 4 test areas (lower legs, forearm and chest) at all time-points (day 7, day 28, day 56, **Figure 3**) compared to baseline. The Composition A effect was major and clinically significant since day 7 (14 applications) with a maximum but milder decrease at day 28 (p<0.0001, Wilcoxon test; intragroup analysis). Decrease of xerosis was maintained on all test areas at day 56.

From day 7 to day 56, scaling measurements on the lower legs initially treated by Composition A showed that MOD and SURFT parameters were no further improved (non significant, Wilcoxon; intragroup analysis), indicating that an optimum effect had been reached during the first 7 days on these 2 parameters, whereas the other parameters SMOD and HI were further improved (p<0.02, Wilcoxon; intragroup analysis; **Figure 1**). On the legs initially treated by vehicle, a more progressive improvement of all parameters were observed, with maximum reduction of scales at day 56 (p<0.006, Wilcoxon, intragroup analysis; no intermediate time point between day 7 and 56).

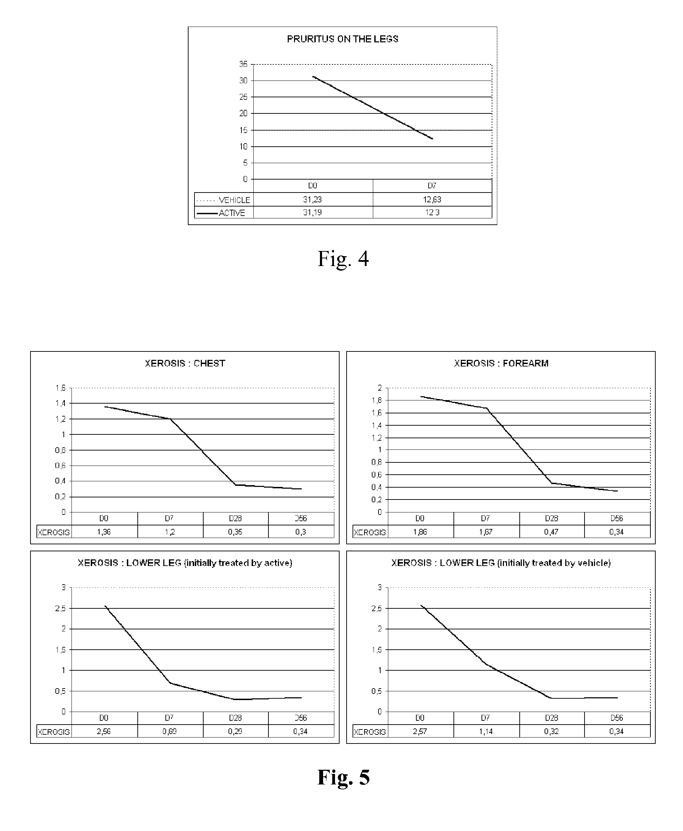

On the basis of both investigators and patient's assessment, Composition A significantly amended global uremic pruritus at all time-points from baseline (p<0.0001, Wilcoxon test; intragroup analysis) **(****Figures 4 and 5****).**
Both DLQI and SF-12 scales demonstrated a marked loss on life quality of the patients at baseline (Mean ± SEM DLQI: 5.61 ± 0.57; SF-12 PCS: 30.51 ± 1.21; SF-12 MCS: 41.29 ± 1.24). At day 56, all scales were improved (DLQI: 2.27 ± 0.46; SF-12 PCS: 33.89 ± 1.34; SF-12 MCS: 46.72 ± 1.28). Differences between baseline and day 56 were statistically significant for all scales (p<0.0001, Wilcoxon test; **Table 6).**

**Table 6: DLQI and SF-12 (PCS and MCS) results at baseline and day 56 in ITT population**

| **Quality of life** | **Baseline (N = 99)** | **Day 56 (N = 99)** |
|---|---|---|
| **DLQI** | | |
| Mean (sem) | 5.61(0.57) | 2.27(0.46) |
| Min / max | 0 / 22 | 0 / 22 |
| median | 3 | 0 |
| **SF-12 PCS** | | |
| Mean (sem) | 30.51 ( 1.21 ) | 33.89 ( 1.34 ) |
| Min / max | 6.5 / 60.64 | 6.5/55.50 |
| Median | 29.60 | 35.11 |
| | | |
| **SF-12 MCS** | | |
| Mean (sem) | 41.29 (1.24) | 46.72 (1.28) |
| Min / max | 18.53/67.46 | 18.53/68.51 |
| Median | 40.16 | 50.72 |

At day 56, 11 patients (11%) had missing data for the parameters global agreement on efficacy and ease of use of the test product. As 10 patients had a score for xerosis superior to 0 (0=smooth skin) in at least one test area, the quotation "not satisfactory at all" was generated for the parameter global agreement on efficacy. Therefore, analysis included 98 patients for global agreement on efficacy and 88 for global agreement on ease of use. At the end of the study, global agreement to the test product by the patients was satisfactory to very satisfactory in 82 patients out of 98 (84%) as regards to efficacy, and in 82 patients out of 88 (93%) for the ease of use.
Among 93 patients, investigators evaluated global local tolerance as very good in 82 patients (88%), good in 7 patients (7.5%), poor in 1 patient (1%) and very poor in 3 patients (3%). Similarly, the global agreement of the test product by the patients as regards to local tolerance was satisfactory to very satisfactory in 84 patients (93%). During the study, 21 adverse events (including 8 serious adverse events) occurred in 19 patients (19%) in the ITT population. Adverse events probably related to the test product were reported in 5 patients (5%). They comprised pruritus (2%), burning sensations (1%), erythema (1%) and algia (1%) following product application. Among them, 2 led to premature study withdrawal. No product-related serious adverse event was reported.

Detailed description and analysis of adverse events that occurred in the study are given in the Clinical Safety Summary.

The results observed in the PP population confirmed those obtained in the ITT population for all the studied parameters.

### Overall conclusion

This clinical study demonstrates that the topical Composition A, which combines glycerol and paraffin (solid and liquid form), is an effective treatment of uremic xerosis of moderate to severe intensity. Its main effect on xerotic lesions was achieved after 7 days, further improved after 28 days, and maintained at day 56. The Composition A effect was clinical, as observed by significant reduction of the clinical grading of the lesions, as well as instrumental (D-Squame®), as shown by the significant decrease of two parameters assessing the extension (SURFT) and the thickness (MOD) of scaling.

Treatment of uremic xerosis by the Composition A was associated with a marked improvement of the quality of life of the patients. Although Composition A significantly decreased the xerosis-associated pruritus (uremic pruritus), no statistical difference between the active group and the vehicle group was shown. This suggests that Composition A excipient on its own is able to amend uremic pruritus.

The acceptability of Composition A by the patients as regards to efficacy, tolerance and ease of use was satisfactory to very satisfactory in the vast majority of patients. The local tolerance profile of the product was good. Test-product related local AE included erythema (1%), pruritus (2%), local burning (1%) and algia (1%). No systemic AE was reported to be probably related to test product.
In conclusion, Composition A showed a good efficacy and safety profile in patients with moderate to severe uremic xerosis.

### References

1. Tsutsumi H, Utsugi, T, Hayashi S. Study on the occlusivity of oil films. J. Soc. Csmet. Chem. 30: 345-356, 1979*.*
2. Marti-Mestres G, Passet J, Maillols H, Van Sam V, Guilhou J.J., Mestres J.P., Guillot B. Evaluation expérimentale de l'hydratation et du pouvoir occlusive in vivo et in vitro d'excipients lipophiles et de leurs émulsions phase huile continue. International Journal of Cosmetic Science 16 : 161-170, 1994*.*
3. Bettinger J, Gloor M, Gehring W. Influence of a pretreatment with emulsions on the dehydration of the skin by surfactants. Int. J. Cosm. Sci. 16 : 53-60, 1994*.*
4. Grunewald AM, Gloor M, Gehring W, Kleesz P. Barrier creams - commercially available creams versus urea - and glycerol- containing oil- in-water emulsions. Dermatosen 43: 69-74, 1995*.*
5. Bettinger J, Gloor M, Peter C, Kleesz P, Fluhr J, Gehring W. Opposing effects of glycerol on the protective function of the horny layer against irritants and on the penetration of hexyl nicotinate. Dermatology 197: 18-24, 1998*.*
6. Fluhr JW, Gloor M, Lehmann L, Lazzerini S, Distante F, Berardesca E. Glycerol accelerates recovery of barrier function in vivo*. Acta. Derm. Venereol. 79: 418-421, 1999*.*
7. Sagiv ae, Dikstein s, Ingber A. The efficiency of humectants as skin moisturizers in the presence of oil. Skin Res. Tech. 7 : 32-35, 2001.
8. Barany E, Lindberg M, Loden M. Unexpected skin barrier influence from nonionic emulsifiers. Int. J. of Pharm. 195:189-195, 2000.

## Claims

1. A pharmaceutical composition comprising a combination of glycerol, white soft paraffin and liquid paraffin as active ingredients for topical use to treat uremic xerosis.

2. Composition according to claim 1 to treat uremic xerosis in patients undergoing maintenance renal dialysis (MRD).

3. Composition according to claim 1 or 2, the white soft paraffin having a dropping point comprised between 35 and 70°C.

4. Composition according to claim 3, the white soft paraffin having a dropping point comprised between 51 and 57°C, preferably about 54°C.

5. Composition according to any of the preceding claims, the white soft paraffin having a consistency comprised between 175 and 195 1/10 mm (cone at 25°C).

6. Composition according to claim 5, the white soft paraffin having a consistency of about 185 1/10 mm (cone penetration at 25°C).

7. Composition according to any of the preceding claims, the white soft paraffin having a viscosity comprised between 4 and 5 cSt at 100°C.

8. Composition according to claim 7, the white soft paraffin having a viscosity of about 4.8 cSt at 100°C.

9. Composition according to any of the preceding claims comprising about 15% of glycerol, about 8% of white soft paraffin and about 2% of liquid paraffin.

10. Composition according to any of the preceding claims comprising one or several excipients selected from the group consisting of stearic acid, glycerol monostearate, polydimethylcyclosiloxane, dimethicone, polyethylene glycol 600, trolamine, propyl parahydroxybenzoate.

11. Composition according to any of the preceding claims in the form of a oil-in-water (O/W) emulsion.
